# EUROPEAN PATENT APPLICATION

(11) **EP 4 717 289 A1**
(43) Date of publication of application: **01.04.2026**
(21) Application number: 24203338.9
(22) Date of filing: 27.09.2024
(51) Int. Cl.: A61M 5/178, A61M 5/28, A61M 5/31, A61M 5/20, A61M 5/50

(54) **SECURING DEVICE AND ARRANGEMENT FOR SECURING AN EMPTY PHARMACEUTICAL AMPULE TO A SYRINGE**

(71) Applicant: Stoffaneller, Martin, Innsbruck (AT)
(72) Inventor: STOFFANELLER, Martin, 6020 Innsbruck (AT); STOFFANELLER, Lorenz, 6020 Innsbruck (AT)
(74) Representative: Gangl, Markus

(57) **Abstract**

A securing device (1) and an arrangement for securing an empty pharmaceutical ampule (2) to a syringe (3) with a viewing area (6) which is arranged such that at least that part of a surface (7) of the empty pharmaceutical ampule (2) which carries an original label can be viewed when the empty pharmaceutical ampule (2) is secured to the empty pharmaceutical ampule (2).

## Description

### TECHNICAL FIELD

The disclosure is in the field of providing a safe way to administer a pharmaceutical preparation to an organism.

### BACKGROUND

There are many ways to administer a pharmaceutical preparation to an organism, e. g. by subcutaneous or intravenous injection.

Mix-up of pharmaceutical preparations is one of the most dangerous and well-known issues in pharmaceutical applications. Despite the well-known risk and established measures to prevent mix-ups they take place far too often with the potential to severely harm patients.

The predominant way to prevent a mix-up of different pharmaceutical preparations is a continuous and consistent relabelling of pharmaceutical preparations in a well-documented way from production to delivery to a human or non-human organism. These labels are sometimes marked by the refilling person or institution. For some pharmaceutical preparations, pharmaceutical industry provides extra labels. These may come together with the pharmaceutical preparation in one package. Sometimes labels (e. g. for an intravenous application by a syringe) are to be peeled off from a pharmaceutical ampulla and must be transferred onto a syringe.

A relabelling of pharmaceutical preparations may be necessary due to refilling of the same for the reason of mixing pharmaceutical preparations (or diluting pharmaceutical preparations which is considered to be a special case of mixing), preparing it from the state of storage to the state of application (e. g. by dissolving), or by refilling it into a bin in use for application into the human body, e. g. by using a syringe.

Relabelling has a multitude of disadvantages:
- Every relabelling step is time consuming; it is costly (a new label is needed), the new label must be made available (the new label must be supplied) and - most important from a quality system point of view - bears the risk of wrong labelling with the risk of a mix-up of pharmaceutical preparations as a consequence.
- Due to relabelling, information passed over might have changed. Mostly information is skipped (due to the lack of space on the label). For example, the batch number and the expiration date may be no longer available for the applicant on the label attached. The loss of information by relabelling is especially of interest when drugs are passed to the next physician during patient transfer including expiration date and lot information.
- Labels and the printed-on information can become unreadable. For example, information can be washed out due to fluids (e. g. rain, secretion).
- The label information could be damaged mechanically, or it could be accidentally peeled off (especially when not sticked properly).

In summary, relabelling is a tedious and high-risk operation.

Before relabelling became dominant, it was accepted to fix ampullas by adhesive tapes to the syringe bodies. This is no longer state of the art for two main reasons:
- Firstly, the opened ampullas' edges have caused several injuries, sometimes cutting the single-use gloves in a hostile and dangerous environment next to a patients' secretions.
- Secondly, the adhesive tapes always covered a part of the volume scale on a syringe which is not acceptable.

The most time-efficient approach administration by injection is the use of prefilled single use syringes. With prefilled syringes a mix-up due to refilling is not possible at all since no refilling is taking place. However prefilled syringes have two major disadvantages:
- Firstly, prefilled syringes are very expensive in comparison to the use of off-the-shelf empty syringes and separate pharmaceutical ampullas.
- Secondly, hermeticity is reduced (compared to glass ampullas) resulting in reduced shelf life.

For these reasons, pharmaceutical industry does not provide prefilled pharmaceutical syringes as an off-the-shelf option for all drugs.

### SUMMARY OF DISCLOSURE

It is an object to provide a securing device and an arrangement which decrease the risk of a mix-up of pharmaceutical preparations.

This object is achieved by a securing device having the features of claim 1 and an arrangement having the features of claim 10.

A securing device for securing an empty pharmaceutical ampule to a syringe, comprises at least one carrier body which has at least:
- at least one first fastener for fastening the empty pharmaceutical ampule to the securing device
- at least one second fastener for fastening a syringe to the securing device
- a viewing area which is arranged such that at least that part of a surface of the empty pharmaceutical ampule which carries an original label can be viewed when the empty pharmaceutical ampule is fastened to the securing device by the at least one first fastener

The securing device is to be used for securing an empty pharmaceutical ampule to a syringe such that at least that part of a surface of the empty pharmaceutical ampule which carries the original label can be viewed when the empty pharmaceutical ampule is fastened to the securing device by the at least one first fastener, the syringe being filled with a pharmaceutical preparation provided by the pharmaceutical ampule.

The securing device allows for the original label of the pharmaceutical ampule to remain in place on the pharmaceutical ampule and to be viewable via the viewing area while the empty pharmaceutical ampule is securely fastened to the securing device via the at least one first fastener and the securing device is securely fastened to the syringe via the at least one second fastener thereby securing the empty pharmaceutical ampule and the syringe relative to each other. Operation of the plunger of the syringe to administer the pharmaceutical preparation is not hindered when the syringe and the empty pharmaceutical ampule are secured to each other via the securing device.

An arrangement in which a syringe and an empty pharmaceutical ampule are secured to each other by a securing device comprises at least:
- a syringe which is filled with a pharmaceutical preparation taken from the pharmaceutical ampule
- a securing device, in particular a securing device as described herein, comprising at least:
   - at least one first fastener for fastening the empty pharmaceutical ampule to the carrier body
   - a viewing area which is arranged such that at least that part of a surface of the empty pharmaceutical ampule which carries the original label can be viewed when the empty pharmaceutical ampule is fastened by the at least one first fastener
- the empty pharmaceutical ampule from which the pharmaceutical preparation has been taken being fastened to the securing device by the at least one first fastener such that at least part of a surface of the empty pharmaceutical ampule carrying an original label describing the pharmaceutical preparation can be viewed via the viewing area

The arrangement is to be used for securing an empty pharmaceutical ampule to a syringe such that at least that part of a surface of the empty pharmaceutical ampule which carries the original label can be viewed when the empty pharmaceutical ampule is fastened to the securing device by the at least one first fastener, the syringe being filled with a pharmaceutical preparation provided by the pharmaceutical ampule.

The arrangement allows for the original label of the pharmaceutical ampule to remain in place on the pharmaceutical ampule and to be viewable via the viewing area while the empty pharmaceutical ampule is securely fastened to the securing device via the at least one first fastener. Operation of the plunger of the syringe to administer the pharmaceutical preparation is not hindered when the syringe and the empty pharmaceutical ampule are secured to each other via the arrangement.

Such a securing device and such an arrangement provide a variety of further advantages, e. g.:
- All information provided by the original label on the empty pharmaceutical ampule is still accessible and readable via the viewing area without the need for any relabelling.
- It is common sense that any work with or next to patients must be performed with single use gloves. However, gloves reduce the tactile sensation. Especially for the work with labels, tactile sense is very advantageous. Getting rid of labels attached to gloves is cumbersome, especially in situations where pharmaceutical preparations must be applied immediately.

This operation is no longer necessary.
- A clean surface on the syringe is no longer needed (preparing a clean surface is often difficult, e. g. because of rainy environment, secretion, powder of gloves). No use of adhesion tape is needed due to the use of a carrier body and the at least one first and/or second fastener.
- In quality-controlled areas a double-check of prepared medication is obligatory for each pharmaceutical preparation. Double-checking is now possible throughout the whole duration of patient treatment.
- The original label is still readable via the viewing area during administration of the empty pharmaceutical preparation.
- The carrier body of the securing device can protect the user from injuries which might be caused by sharp edges of the empty pharmaceutical ampule.
- A volume scale provided on the syringe remains readable as it is not covered by a label which has been removed from the empty pharmaceutical ampule or another repository of labels and attached to the syringe.
- It can be provided that a total length of the securing device along a longitudinal extension of the securing device is substantially equal to or smaller than a total length of the syringe and/or the pharmaceutical ampule allowing for a compact design of the securing device.

It is to be understood that the term "empty" pharmaceutical ampule does not require that all of the pharmaceutical preparation has been taken out of the pharmaceutical ampule but encompasses situations where some pharmaceutical preparation remains in the pharmaceutical ampule.

### DESCRIPTION OF EMBODIMENTS

In some embodiments the viewing area is formed by a construction of the securing device which leaves at least that part of the empty pharmaceutical ampule uncovered which carries the original label. In order to protect the original label, the securing device might comprise a transparent cover for the original label.

In some embodiments the at least one first fastener and/or the at least one second fastener comprise(s) at least one clamp. Preferably a clamp which snap-on functionality is used as no operation of a latch mechanism is needed. The use of clamps allows for easy production and handling of the securing device. For example, one of the clamps can serve to fasten the securing device to the syringe and another one of the clamps can serve to fasten the empty pharmaceutical ampule to the securing device. As an alternative to a clamp a strap could be used which would have the advantage that it would work with syringes and/or pharmaceutical ampules of different sizes.

In some embodiments the securing device comprises a carrier body formed of a rigid material, preferably rigid plastic. Such a securing device can be manufactured by injection moulding.

In some embodiments the carrier body has one part with a longitudinal extension along which the syringe and the empty pharmaceutical ampule can be aligned, and which carries at least part of the first and/or second fastener.

In some embodiments, in particular in those of the preceding paragraph, one part of the carrier body can be provided which is near the injection opening of the syringe, e. g. having the shape of a thick or thin plate and/or being arranged at one end of the part having the longitudinal extension.

In some embodiments, in particular in those of one of the two preceding paragraphs, a finger flange can be arranged.

In some embodiments the at least one first fastener and/or the at least one second fastening form(s) part of the carrier body. This allows for the production of the securing device in one step as a whole, e. g. using injection moulding.

In some embodiments the securing device comprises at least one storage space for pharmaceutical labels. In these embodiments a plurality of pharmaceutical labels could be stored and the correct label (with respect to the pharmaceutical ampule which has been chosen) can be selected and arranged such that in addition to the original label of the empty pharmaceutical ampule which can be seen via the viewing area the selected label can be seen.

In some embodiments the arrangement of selectable pharmaceutical labels could be predetermined by the applicant to match the repository of pharmaceutical ampules to labels available in the storage space for pharmaceutical labels.

In some embodiments at least one label can be separated from the labels in the storage space for pharmaceutical labels by a label lock.

In some embodiments the securing device comprises at least one battery compartment. Providing at least one battery allows for a variety of functions which need energy as described in the following embodiments. The functions powered by the battery can be activated either by inserting a battery into the battery compartment or, in some embodiments, by using a dedicated power switch.

In some embodiments the securing device comprises at least one computing device which is configured, e. g., to alert a user about pre-defined conditions such as when a temperature of the pharmaceutical preparation is outside a given range.

In some embodiments the securing device comprises at least one light source. This allows for easier reading especially in dark environments or during night.

In some embodiments the securing device comprises at least one sensor. The at least one sensor can be used to measure a temperature of the pharmaceutical preparation or analyse blood.

In some embodiments the securing device comprises at least one magnet. By way of the at least one magnet the securing device can be attached to a magnetic holder, e. g. of the kind which can be worn on the body of a user. Alternatively, or in addition a hook-and-loop fastener or another snap-on fastener could be used.

In some embodiments the securing device comprises a holder for a cap of the syringe. This allows quick and safe storing of the cap after it has been removed from the syringe.

In some embodiments the securing device comprises an ultrasonic coupler which can be used to mix a pharmaceutical preparation in the form of a dispersion.

In some embodiments the securing device comprises a communication interface which can be used, e. g., to communicate information about temperature, fill level, and so on, to an external device or could be used to operate a finding function.

In some embodiments the securing device comprises a temperature control device which allows for controlling a temperature (e. g. by way of cooling and/or heating) of a pharmaceutical preparation.

In some embodiments the at least one first fastener and the at least one second fastener are arranged relative to each other such that an empty ampule and a syringe fastened to the carrier body are arranged coaxially along a part of the carrier body having a longitudinal extension.

In some embodiments the securing device comprises a scaler doubler in the form of a stick that extends a syringe in an axis parallel to a piston.

In some embodiments the securing device is sterilized and arranged inside a sterile package.

In some embodiments of the arrangement the securing device comprises at least one second fastener for fastening the securing device to a syringe.

In some embodiments of the arrangement the securing device is formed as a body of material which body also forms the syringe and the at least one first fastener and the viewing area. In these embodiments it is not necessary to use an extra syringe as the carrier body already forms a syringe. For this reason, no second fastener is needed. It is only necessary to fasten the empty pharmaceutical ampule to the securing device.

In some embodiments of the arrangement the securing device has the form of a plunger which is arranged in the syringe and wherein the at least one first fastener forms part of the plunger. For example, the plunger can have a space in which the empty pharmaceutical ampule can be securely arranged.

In some embodiments of the arrangement, it is sterilized and arranged inside a sterile package.

In some embodiments the at least one fastener device is made of transparent material and the viewing area is formed by a transparent region of the at least one fastener device.

In some embodiments the at least one fastener device is made of material that is compatible with sterilisation.

In a preferred embodiment the securing device for securing an empty pharmaceutical ampule to a syringe has at least:
- a carrier body, which is preferably in the form of a single piece of material and/or which is preferably made of plastic
- a first fastener, which is preferably formed together with the one carrier body as a single body, for fastening the empty pharmaceutical ampule to the carrier body
- a second fastener, which is preferably formed together with the one carrier body as a single body, for fastening the carrier body to a syringe
wherein
- the first fastener comprises at least one clamp and preferably an opening in the carrier body such that the empty pharmaceutical ampule can be inserted into the opening and can be snapped onto the securing device via the at least one clamp (the optional opening and the at least one clamp being arranged spaced from each other along a longitudinal extension of the carrier body)
- the second fastener comprises at least two clamps (at least an upper clamp and a lower clamp along the longitudinal axis of the carrier body) with snap-in functionality.
- the securing device has a viewing area which is arranged such that at least that part of a surface of the empty pharmaceutical ampule which carries the original label can be viewed when the empty pharmaceutical ampule is fastened to the securing device by the at least one first fastener
- optionally, the first fastener and the second fastener are arranged on opposing sides of a part of the carrier body which part extends along the longitudinal extension

### BRIEF DESCRIPTION OF DRAWINGS

Figure 1 shows an embodiment of the securing device together with an empty pharmaceutical ampule and a syringe.
Figure 2 shows another embodiment of the securing device together with an empty pharmaceutical ampule and a syringe.
Figure 3 shows an arrangement using the embodiment of Figure 2 wherein a syringe and an empty pharmaceutical ampule have been fastened to the securing device and have thereby been secured to each other.
Figure 4 shows another embodiment of the arrangement in which the empty pharmaceutical ampule is fastened to a security device in the form of a plunger.
Figure 5 shows a schematic view of an embodiment of the securingdevice having a variety of functional elements, all of which or only some of which or none can be present in a securing device.

### DETAILED DESCRIPTION OF DRAWINGS

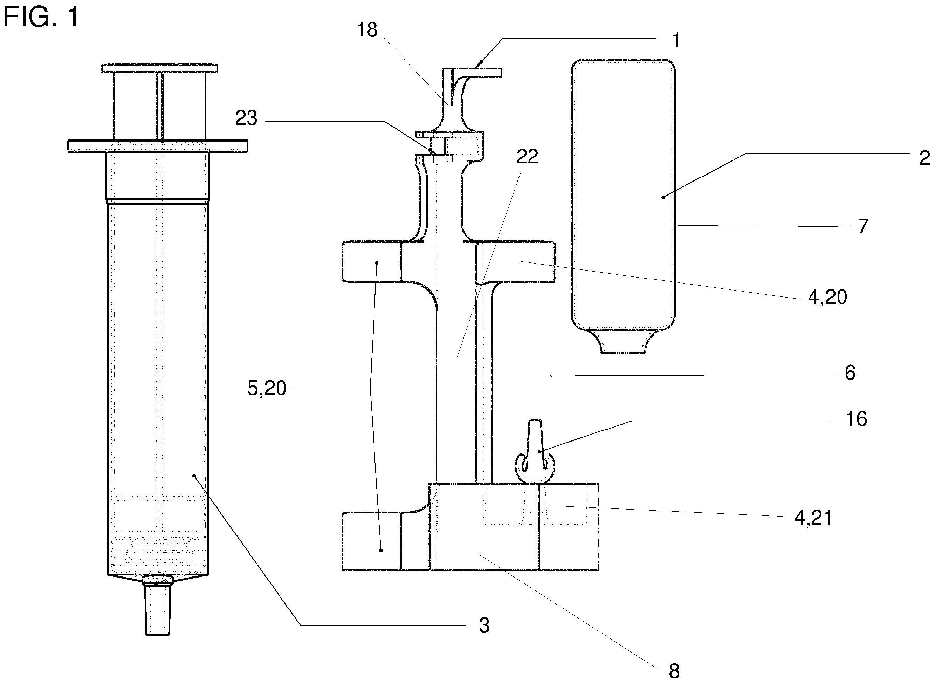

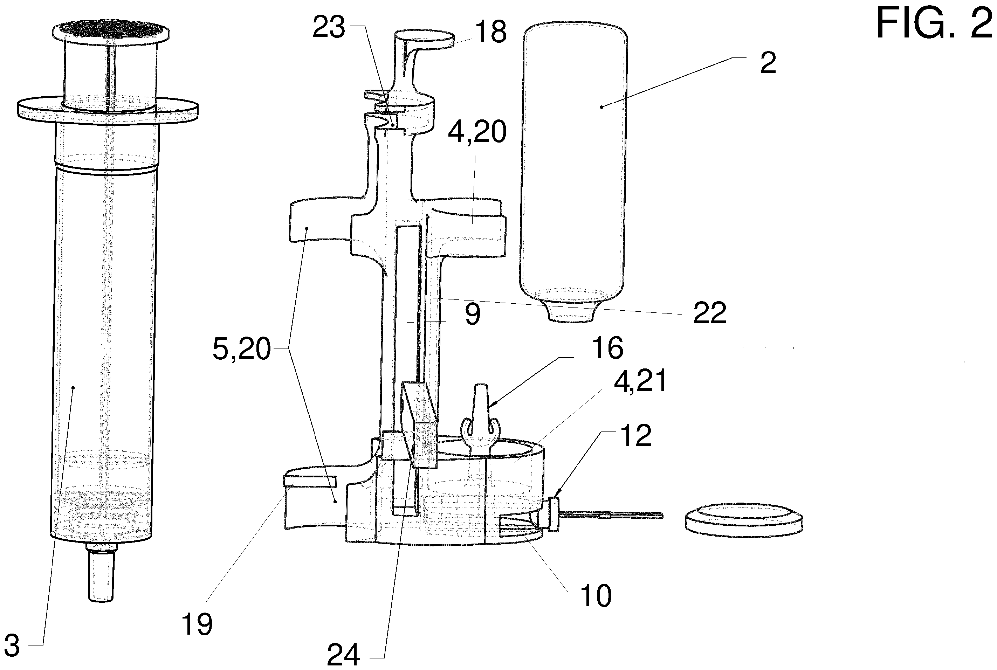

The embodiments of a securing device 1 and an arrangement shown in Figures 1 and 2 is of the type wherein the securing device 1 is in the form of a unitary carrier body 8 which carries a first fastener 4 and a second fastener 5.

The carrier body 8 has one part 22 with a longitudinal extension along which the syringe 3 and the empty pharmaceutical ampule 2 can be aligned by fastening to at least part of the first and/or second fastener 4, 5.

At one end of the part 22 having the longitudinal extension a part having the shape of a thick plate is arranged forming that part of the securing device 1 which is near the injection opening of the syringe 3. Alternatively, a thin plate (in particular, if no components are to be arranged inside the plate) or a differently shaped body could be used.

At the other end of the part 22 having the longitudinal extension and at the side to which the syringe 3 is to be fastened a finger flange is arranged.

The first fastener 4 comprises a single clamp 20 and an opening 21 in the carrier body 8 such that the empty pharmaceutical ampule 2 can be inserted into the opening 21 and can be snapped onto the securing device 1 via the upper clamp 20.

The second fastener 5 comprises two clamps 20 with snap-in functionality which are arranged spaced from each other along a longitudinal axis of part 22 of the carrier body 8. The flange fit 23 prevents unintentional twisting along the longitudinal extension.

The stud 19 holds the pharmaceutical labels 24 in place and serves as hinge for at least one label to be released out of the storage space for pharmaceutical labels. The label lock 27 keeps the selected at least one label in place.

Although as shown there the second fasteners 5 comprises two clamps 20 and the first fastener 4 comprises a single clamp 20, different numbers of clamps 20 for the first fastener 4 and/or the second fastener 5 could be used.

In the embodiment of Figure 2 the carrier body 8 optionally also carries
- a holder for a cap of a syringe 3
- a scaler doubler in the form of a stick 18 that extends a syringe 3 in axis parallel to a piston in the direction of a thumb rest
- a battery compartment 10 for a battery (in the part having the shape of a thick plate)
- storage space 9 for pharmaceutical labels (in the part 22 having the longitudinal extension), in which a plurality of pharmaceutical labels is arranged such that they can be pivoted out of the storage space 9, wherein one of the pharmaceutical labels has been pivoted out of the storage space 9 such that it can be read
- a light source 12 (in the part having the shape of a thick plate) to be energized by the battery, in particular a source of redlight which is preferable for night vision

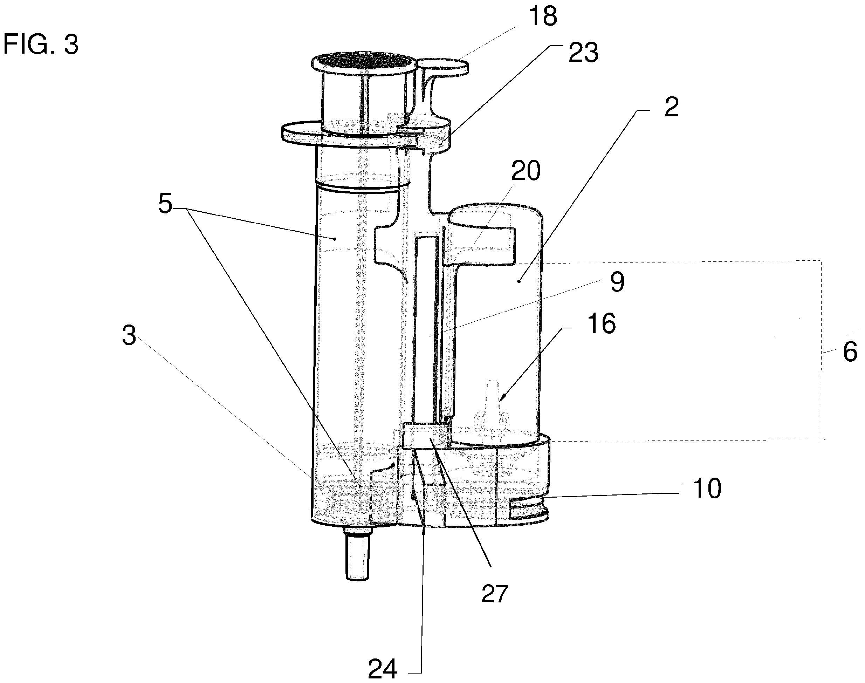

In Figure 1 and Figure 2 the syringe 3 and the empty pharmaceutical ampule 2 are not yet fastened to each other via the securing device 1 but this situation is shown in Figure 3 for the embodiment of Figure 2. Figure 2 is an exploded view of Figure 3.

As can be seen from Figure 3 the whole area of the surface 7 of the empty pharmaceutical ampule 2 between the clamp 20 of the first fastener 4 and an upper surface of a lower part of the carrier body 8 which has the opening 21 is visible when the empty pharmaceutical ampule 2 is fastened by the first fastener 4 and therefore forms the viewing area 6.

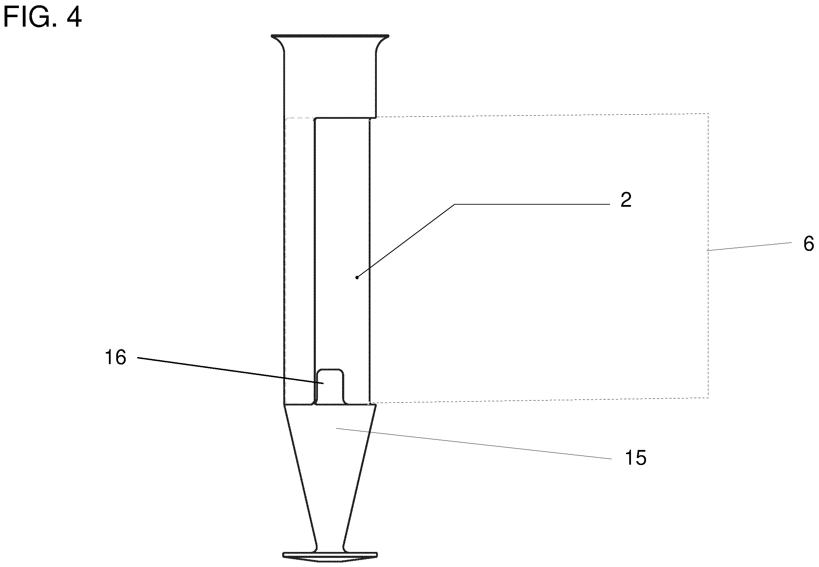

Figure 4 shows another embodiment of the arrangement with a securing device 1 in the form of a plunger 15 into which an empty pharmaceutical ampule 2 has been inserted. The plunger 15 is provided with an opening which forms the viewing area 6.

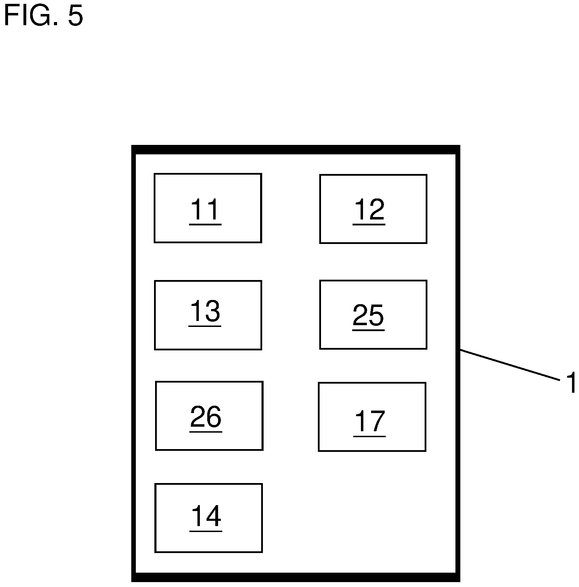

Figure 5 shows a schematic view of another embodiment of the securing device 1. In this embodiment the securing device 1 is provided with:
- a storage space 9 for pharmaceutical labels (not shown in this Figure)
- a battery compartment 10 (not shown in this Figure)
- a computing device 11
- a light source 12
   - -a sensor 13
- a magnet 14
- a holder for a cap of the syringe 3 (not shown in this Figure)
- an ultrasonic coupler 25
- a communication interface 26
- a temperature control device 17

The usual connections between the functional elements are not shown.

### LIST OF REFERENCE NUMBERS

- 1: securing device
- 2: empty pharmaceutical ampule
- 3: syringe
- 4: first fastener
- 5: second fastener
- 6: viewing area
- 7: surface of the empty pharmaceutical ampule
- 8: carrier body
- 9: storage space for pharmaceutical labels
- 10: battery compartment
- 11: computing device
- 12: light source
- 13: sensor
- 14: magnet
- 15: plunger
- 16: thorn
- 17: temperature control device
- 18: stick
- 19: stud
- 20: clamp
- 21: opening
- 22: part of the carrier body having a longitudinal extension
- 23: flange fit
- 24: extra label
- 25: ultrasonic coupler
- 26: communication interface
- 27: label lock

## Claims

1. A securing device (1) for securing an empty pharmaceutical ampule (2) to a syringe (3), comprising at least one carrier body (8) which has at least:
- at least one first fastener (4) for fastening the empty pharmaceutical ampule (2) to the securing device (1)
- at least one second fastener (5) for fastening a syringe (3) to the securing device (1)
- a viewing area (6) which is arranged such that at least that part of a surface (7) of the empty pharmaceutical ampule (2) which carries an original label can be viewed when the empty pharmaceutical ampule (2) is fastened to the securing device (1) by the at least one first fastener (4).

2. The securing device of the preceding claim wherein the carrier body (8) has one part (22) with a longitudinal extension which carries at least part of the first and/or second fastener (4, 5) wherein the syringe (3) and the empty pharmaceutical ampule (2) are aligned along this part (22) when being fastened to the carrier body (8).

3. The securing device of one of the preceding claims wherein the carrier body (8) has one part which is near an injection opening of the syringe (3), preferably having the shape of a plate and/ or being arranged at one end of the part (22) having the longitudinal extension.

4. The securing device of at least one of the preceding claims wherein the at least one first fastener (4) and/or the at least one second fastening (5) comprise(s) at least one clamp (20), preferably with snap-on functionality.

5. The securing device of at least one of the preceding claims wherein carrier body (8) is formed of a rigid material, preferably rigid plastic.

6. The securing device of at least one of the preceding claims wherein the at least one first fastener (4) forms a single part of the carrier body (8) and/or the at least one second fastener (5) forms a single part of the carrier body (8).

7. The securing device of at least one of the preceding claims wherein the securing device (1) comprises at least one of the following:
- at least one storage space (9) for pharmaceutical labels
- at least one battery compartment (10)
- at least one computing device (11) which is configured to
- at least one light source (12)
- at least one sensor (13)
- at least one magnet (14)
- a holder for a cap of the syringe (3)
- an ultrasonic coupler (25)
- a communication interface (26)
- a temperature control device (17)
- a flange fit (23)

8. The securing device of at least one of the preceding claims wherein the at least one first fastener (4) and the at least one second fastening (5) are arranged relative to each other such that an empty pharmaceutical ampule (2) and a syringe (3) fastened to the carrier body (8) are arranged coaxially along a part (22) of the carrier body (8) having a longitudinal extension.

9. The securing device of at least one of the preceding claims wherein the securing device (1) comprises a scaler doubler in the form of a stick (18) that extends a syringe (3) in an axis parallel to a piston.

10. An arrangement in which a syringe (3) and an empty pharmaceutical ampule (2) are secured to each other by a securing device (1), comprising at least:
- a syringe (3) which is filled with a pharmaceutical preparation taken from the pharmaceutical ampule (2)
- a securing device (1), in particular a securing device (1) according to at least one of the preceding claims, comprising at least a carrier body (8) with:
• at least one first fastener (4) for fastening the empty pharmaceutical ampule (2) to the carrier body (8)
• a viewing area (6) which is arranged such that at least that part of a surface (7) of the empty pharmaceutical ampule (2) which carries the original label can be viewed when the empty pharmaceutical ampule (2) is fastened by the at least one first fastener (4)
- the empty pharmaceutical ampule (2) from which the pharmaceutical preparation has been taken being fastened to the securing device (1) by the at least one first fastener (4) such that at least that part of a surface (7) of the empty pharmaceutical ampule (2) carrying an original label describing the pharmaceutical preparation can be viewed via the viewing area (6).

11. The arrangement of claim 10 wherein the securing device (1) comprises at least one second fastener (5) for fastening the securing device (1) to a syringe (3).

12. The arrangement of claim 10 wherein the securing device (1) is formed as a body of material which body also forms the syringe (3) and the at least one first fastener (4) and the viewing area (6).

13. The arrangement of claim 10 wherein the securing device (1) has the form of a plunger (15) which is arranged in the syringe (3) and wherein the at least one first fastener (4) forms part of the plunger (15).

14. The securing device (1) according to at least one of claims 1 to 9 or the arrangement of at least one of claims 10 to 13 wherein the securing device (1) and the arrangement, respectively, is sterilized and arranged inside a sterile package.

15. Use of a securing device (1) according to at least one of claims 1 to 9 or of an arrangement according to at least one of claims 10 to 14 for securing an empty pharmaceutical ampule (2) to a syringe (3) such that at least that part of a surface (7) of the empty pharmaceutical ampule (2) which carries the original label can be viewed when the empty pharmaceutical ampule (2) is fastened to the securing device (1) by the at least one first fastener (4), the syringe (3) being filled with a pharmaceutical preparation provided by the pharmaceutical ampule (2).
